# EUROPÄISCHE PATENTSCHRIFT

(11) **EP 0 546 419 B1**
(45) Veröffentlichungstag und Bekanntmachung des Hinweises auf die Patenterteilung: **28.02.1996**
(21) Anmeldenummer: 92120436.8
(22) Anmeldetag: 30.11.1992
(51) Int. Cl.: C07C 13/28, C09K 5/00

(54) **Wärmeträgerflüssigkeit und Verfahren zu ihrer Herstellung**
Heat transfer liquid and its preparation
Liquide de transfer de chaleur et sa préparation

(30) Priorität: 13.12.1991 DE 4141191
(43) Veröffentlichungstag der Anmeldung: 16.06.1993
(73) Patentinhaber: BAYER AG, D-51368 Leverkusen (DE)
(72) Erfinder: Klein, Alfons, Dipl.-Ing., W-4000 Düsseldorf 1 (DE); Kron, Rudolf, Dipl.-Ing., W-5303 Bornheim 2 (DE); Fiege, Helmut, Dr., W-5090 Leverkusen 1 (DE); Paetz, Klaus-Christian, Dr., W-5093 Burscheid (DE)

(56) Entgegenhaltungen:
- FR-A- 2 138 838
- US-A- 1 908 190
- US-A- 2 233 964
- US-A- 3 786 106
- US-A- 3 793 383
- DATABASE WPI Week 7414, Derwent Publications Ltd., London, GB; AN 74-25754
- DATABASE WPI Week 7420, Derwent Publications Ltd., London, GB; AN 74-37293

## Beschreibung

Die Erfindung betrifft bei Raumtemperatur flüssige Mischungen aus A.-C. verschiedenen Cyclohexylbiphenylen und D. Dicyclohexylbenzolen und/oder Phenylbicyclohexylen und/oder Tercyclohexylen und gegebenenfalls höheren polycyclischen Kohlenwasserstoffen, Verfahren zu ihrer Herstellung und ihre Verwendung als Wärmeträger.

"Wärmeträger" im Sinne der Erfindung sind Wärmeübertragungsmittel, die beispielsweise in Kühl- und Heizkreisläufen und in Wärmerückgewinnungsanlagen verwendet werden. Von solchen Wärmeträgern erwartet man u.a. eine hohe Wärmekapazität, hohe thermische Belastbarkeit und vor allem eine möglichst niedrige Viskosität, weil die Viskosität die erforderliche Pumpenleistung (und damit die Eignung des Wärmeträgers für die Verwendung bei niedrigen und hohen Temperaturen) und die Strömungsgeschwindigkeit (und damit den erreichbaren Wärmeübergang und die Dimensionierung der Wärmeaustauscherflächen) mitbestimmt. Bei der Verwendung der Wärmeträger zu Kühlzwecken wird durch die Viskosität die niedrigste mögliche Einsatztemperatur festgelegt.

Wegen ihres chemisch inerten Verhaltens auch bei hohen Temperaturen waren in der Vergangenheit chlorierte Polyphenyle die beliebtesten Wärmeträger. Nachdem man chlorierte Polyphenyle aus den bekannten Gründen für diesen Zweck nicht mehr einsetzt, ist man auf nicht-halogenierte Polyphenyle und ihre Derivate, wie z.B. partiell hydrierte Terphenyle, ausgewichen (Firmenschrift Monsanto, Wärmeträgerflüssigkeiten, Einführungs-Ausgabe 1991, S. 4).

Terphenyle fallen bei der Biphenyl-Herstellung als Nebenprodukte an. Da die thermische Biphenyl-Synthese - die pyrogene Benzol-Dehydrierung bei 700 bis 800°C - zur weitgehenden Vermeidung von Abbauprodukten (Ruß!) in Gegenwart von Schwefelkohlenstoff durchgeführt wird, enthalten das resultierende Diphenyl und die als Nebenprodukte anfallenden Terphenyle geringe Mengen Schwefelverbindungen, die bekanntlich als Katalysatorgifte wirken und daher die Terphenyl-Hydrierung erschweren. Ein technisch fortschrittliches Herstellungsverfahren sollte diesem Problem Rechnung tragen.

Es wurde nun gefunden, daß Mischungen aus teilweise und völlig hydrierten Terphenylen der anspruchsgemäßen Art die geforderten vorteilhaften Wärmeträger-Eigenschaften besitzen.

Gegenstand der Erfindung sind also Mischungen aus
A. 4 bis 30, vorzugsweise 10 bis 25 Gew.-% 2-Cyclohexylbiphenyl,
B. 1 bis 15, vorzugsweise 3 bis 9 Gew.-% 3-Cyclohexylbiphenyl,
C. 4 bis 30, vorzugsweise 11 bis 20 Gew.-% 4-Cyclohexylbiphenyl und
D. 45 bis 65 Gew.-% Dicyclohexylbenzolen und/oder Phenylbicyclohexylen und/oder Tercyclohexylen und gegebenenfalls höheren polycyclischen Kohlenwasserstoffen,
wobei sich die Prozentangaben jeweils auf die Summe der Komponenten A bis D beziehen.

Die obigen erfindungsgemäßen Mischungen lassen sich durch Hydrierung von Gemischen aus Cyclohexylbiphenylen und Terphenylen herstellen.

Cyclohexylbiphenyle hat man in der Vergangenheit durch Umsetzung von Biphenyl mit Cyclohexen oder Halogencyclohexan in Gegenwart typischer Friedel-Crafts-Katalysatoren, wie beispielsweise Aluminiumchlorid, hergestellt (JP 1973/92 347 vom 30.11.1973; JP 1974/5949 vom 19.1.1974; JP 1974/11 860 von 1.2.1974). Bei der Verwendung von Aluminiumchlorid treten allerdings Korrosionsprobleme bei den verwendeten Reaktoren auf. Es hat sich nun überraschenderweise herausgestellt, daß sich Cyclohexylbiphenyle in guter Ausbeute durch Umsetzung von Biphenyl mit Cyclohexanol in Gegenwart von Bleicherde bilden, was einer Friedel-Crafts-Alkylierung von Biphenyl mit dem aus Cyclohexanol entstehenden Cyclohexen entspricht.

Die Erfindung macht daher auch ein Verfahren zur Herstellung von Cyclohexylbiphenylen aus Biphenyl und Cyclohexanol in Gegenwart von 0,3 bis 5, vorzugsweise 0,8 bis 2 Gew.-% Bleicherde, bezogen auf eingesetztes Biphenyl, zugänglich.

Dabei kann das Molverhältnis der Ausgangsprodukte Biphenyl und Cyclohexanol innerhalb weiter Grenzen schwanken; es beträgt im allgemeinen 1 bis 5, vorzugsweise 2 bis 3.

Die Reaktionstemperatur beträgt im allgemeinen 140 bis 220°C, vorzugsweise 160 bis 190°C.

Unter "Bleicherden" versteht man feinteilige Materialien, die sich im wesentlichen von Aluminium- und/oder Siliciumoxiden - vorzugsweise solchen mit Band- oder Blattstruktur - ableiten, Bevorzugte Bleicherden umfassen überwiegend Aluminosilikate wie die Montmorillonit- Gruppe, zu der Bentonite, Nontronite, Baydellite und Hectorite, aber auch Kaoline zählen, sowie im wesentlichen Quarz-ähnliche Produkte wie Kieselgur und verwandte Sedimente. Solche Bleicherden können ohne Vorbehandlung eingesetzt werden; vorzugsweise werden sie aber einer Behandlung mit Säure, insbesondere mit Mineralsäure wie Schwefelsäure, Phosphorsäure, Salzsäure, Perchlorsäure oder Fluorwasserstoffsäure unterworfen. Solche "aktivierten" Bleicherden werden beispielsweise in Ullmanns Encyclopädie der technischen Chemie, 3. Auflage, Band 4, Seite 541 ff. (1953), Band 8, Seiten 801 bis 804 (1957) und Band 9, Seite 271 ff. (1957), Urban & Schwarzenberg, München-Berlin, beschrieben.

Man kann erfindungsgemäß beispielsweise so arbeiten, daß man Biphenyl und Bleicherde vorlegt und bei erhöhter Temperatur Cyclohexanol zuführt. Mit dem zunächst entstehenden Cyclohexen kann das entstehende Reaktionswasser kontinuierlich abdestilliert und mit einem Wasserabscheider abgetrennt werden. Das zurückgeführte Cyclohexen dient dann zur Alkylierung des Biphenyls. Zur Aufarbeitung kann das Reaktionsgemisch heiß vom Katalysator abgesaugt werden. Anschließend wird man überschüssiges Biphenyl abdestillieren. Der Destillationsrückstand, der im wesentlichen aus Cyclohexylbiphenylen besteht, kann ohne weitere Reinigungsschritte verarbeitet oder durch Destillation gereinigt werden.

Es versteht sich von selbst, daß bei der Herstellung der Cyclohexylbiphenyle als Nebenprodukte auch höhere polycyclische Kohlenwasserstoffe mit mehr als 3 Ringen pro Molekül (z.B. Dicyclohexylbiphenyle) entstehen können (wobei die einzelnen Ringe dieser Polycyclen kein gemeinsames Ringatom und keine gemeinsame Ringbindung besitzen). Die Menge dieser höheren polycyclischen Nebenprodukte wird 20 Gew.-%, vorzugsweise 15 Gew.-% des Biphenyl-freien Reaktionsproduktes der Cyclohexylbiphenyl-Herstellung nicht überschreiten. Sofern nicht die einzelnen Isomeren (z.B. "2-Cyclohexylbiphenyl") genannt sind, meint der Ausdruck "Cyclohexylbiphenyl" im Sinne der Erfindung das Reaktionsprodukt der Cyclohexylbiphenyl-Herstellung mitsamt den als Nebenprodukten entstandenen höheren polycyclischen Kohlenwasserstoffen.

Weiterhin wurde überraschenderweise gefunden, daß mit Bleicherde auch die weiter oben geschilderte Terphenyl-Problematik zufriedenstellend gelöst werden kann:
Wenn man auf das für die Hydrierung vorgesehene Gemisch aus Terphenylen und Cyclohexylbiphenylen Bleicherde einwirken läßt und diese dann abtrennt, erhält man Produkte, die sich (verglichen mit unbehandelten Gemischen) bedeutend schneller hydrieren lassen. Das Abtrennen der Bleicherde bei Ansätzen in großtechnischem Maßstab bereitet wegen des hohen Schmelzpunkts des Gemischs Schwierigkeiten. Wesentlich einfacher verläuft die Reaktion, wenn man das Gemisch mit zusätzlichem Biphenyl verdünnt. Ein solches Gemisch läßt sich im Zusammenhang mit der Synthese von Cyclohexylbiphenylen herstellen:
Nach der Umsetzung von überschüssigem Biphenyl mit Cyclohexanol in Gegenwart von Bleicherde versetzt man das Reaktionsgemisch mit rohem Terphenyl und rührt 0,5 bis 4 Stunden bei 130 bis 160°C. Danach kann man die Bleicherde vom heißen Gemisch absaugen, das überschüssige Biphenyl abdestillieren und den Rückstand der Hydrierung zuführen. Dieser Rückstand läßt sich in etwa der gleichen Zeit wie ein Gemisch aus Cyclohexylbiphenylen und schwefelfreien Terphenylen hydrieren.

Weiterer Gegenstand der Erfindung ist also ein Verfahren zur Herstellung der Mischungen gemäß Anspruch 1 durch partielle Hydrierung eines Gemisches aus Terphenylen und Cyclohexylbiphenylen, dadurch gekennzeichnet, daß die für die Hydrierung eingesetzte Mischung oder ihre Komponenten mit Bleicherde behandelt worden ist/sind.

Entsprechend den obigen Ausführungen über die in den Cyclohexylbiphenylen als Nebenprodukte eventuell enthaltenen höheren polycyclischen Kohlenwasserstoffe können auch die für die Hydrierung vorgesehenen Gemische aus Terphenylen und Cyclohexylbiphenylen diese höheren polycyclischen Kohlenwasserstoffe enthalten; auch die Hydrierungsprodukte können diese höheren polycyclischen Kohlenwasserstoffe beziehungsweise ihre Hydrierungsprodukte enthalten.

Die Hydrierung erfolgt in der Regel bei mäßig erhöhtem Druck und bei erhöhter Temperatur in Gegenwart von 1 bis 10, vorzugsweise 3 bis 6 Gew.-% eines Hydrierkatalysators, beispielsweise eines Nickelkatalysators. Im allgemeinen wird feinverteiltes metallisches Nickel auf einem Träger oder in Form von Raney-Nickel eingesetzt. Die Hydrierung kann abgebrochen werden, wenn der Anteil D der anspruchsgemäßen Mischungen im Bereich von 45 bis 65 Gew.-%, bezogen auf die Summe der Komponenten A bis D, liegt.

Aromaten kann man bekanntlich nur unter forcierten Bedingungen hydrieren. Für Aromaten-Kern-Hydrierungen werden im allgemeinen Drucke von mindestens 100 bar und Temperaturen von über 250°C angewandt (J. Org. Chem., Vol. 30 (1965), S. 384-388; Ullmanns Encyclopädie der technischen Chemie, 4. Aufl., Bd. 9. S. 683, Urban & Schwarzenberg, München-Berlin 1975). Es war daher über- aus überraschend, daß die Hydrierung eines Gemisches aus Terphenylen und Cyclohexylbiphenylen bei relativ milden Bedingungen erfolgen kann. Statt aufwendiger Hochdruckautoklaven können somit nun normale Reaktoren eingesetzt werden.

Weiterer Gegenstand der Erfindung ist daher ein Verfahren zur Herstellung von Mischungen gemäß Anspruch 1 durch partielle Hydrierung eines Gemisches aus Terphenylen und Cyclohexylbiphenylen in Gegenwart eines Hydrierkatalysators, dadurch gekennzeichnet, daß man die Hydrierung bei Temperaturen von 120 bis 190°C, vorzugsweise von 160 bis 180°C, und bei Drucken von 2 bis 25, vorzugsweise 8 bis 15 bar durchführt.

Für die Art des geeigneten Katalysators und das Ende der Hydrierung gelten die weiter oben gemachten Angaben.

Aufgabe der Erfindung war auch das Bereitstellen eines Gesamtkonzepts für die Herstellung eines modernen Wärmeträgers ausgehend von Biphenyl und Terphenyl auf möglichst wirtschaftlicher Basis. Die Durchführung der geschilderten Verfahren stellt ein solches von Biphenyl und Terphenyl ausgehendes Konzept dar, das zu den in Anspruch 1 definierten Mischungen führt.

Weiterer Gegenstand der Erfindung ist also ein Verfahren zur Herstellung von Mischungen gemäß Anspruch 1, wonach man Biphenyl und Cyclohexanol im Molverhältnis (1 bis 5):1 in Gegenwart von 0,3 bis 5 Gew.-%, bezogen auf eingesetztes Biphenyl, Bleicherde auf eine Temperatur von 140 bis 220°C erhitzt, wobei das freiwerdende Wasser zusammen mit einem Teil des entstandenen Cyclohexen azeotrop entfernt und das Cyclohexen rückgeführt wird, dem entstandenen Reaktionsgemisch das 0,5 bis 1,2-fache der im Reaktionsgemisch befindlichen Cyclohexylbiphenyle an Terphenyl und so viel Biphenyl zusetzt, daß der Anteil Biphenyl im Rohgemisch (nach der Terphenylzugabe) mindestens 30 Gew.-% beträgt, und die resultierende Mischung bei einer Temperatur von 130 bis 160°C 0,5 bis 4 Stunden rührt, die Bleicherde und Biphenyl abtrennt, den Rückstand in Gegenwart eines Hydrierkatalysators bei einer Temperatur von 120 bis 190°C und einem Druck von 2 bis 25 bar hydriert, bis der Anteil D der anspruchsgemäßen Mischungen im Bereich von 45 bis 65 Gew.-%, bezogen auf die Komponenten A bis D, liegt, den Hydrierkatalysator abtrennt und das verbleibende Reaktionsgemisch destilliert.

Die erfindungsgemäßen Mischungen besitzen eine kinematische Viskosität von 80 bis 90 mm²/sec bei 20°C. Aufgrund der hohen Siedepunkte der einzelnen Komponenten eignen sich die erfindungsgemäßen Mischungen hervorragend als Wärmeträger in unter Normaldruck stehenden Systemen.

Weiterer Gegenstand der Erfindung ist die Verwendung der erfindungsgemäßen Mischungen als Wärmeträger.

Die Prozentangaben in den nachfolgenden Beispielen beziehen sich jeweils auf das Gewicht; Teile sind Gewichtsteile.

### Beispiele

### Beispiel 1

### A) Herstellung von Cyclohexylbiphenylen

In einem 2 l Dreihalskolben mit Wasserabscheider werden 981,6 g Biphenyl und 12,5 g Bleicherde (®Tonsil KSFO der Firma Südchemie, mit Schwefelsäure aktivierte Bleicherde) vorgelegt. Bei 160°C läßt man innerhalb von 5 Stunden 214 g Cyclohexanol eintropfen` Das entstandene Reaktionswasser (ca. 36 ml) geht mit etwas zwischenzeitlich gebildetem Cyclohexen azeotrop über und wird ausgekreist, Man läßt 5 Stunden bei 180°C nachrühren und saugt anschließend bei 100 bis 120°C ab, In der folgenden Destillation über eine 30 cm Kolonne wird überschüssiges Biphenyl abdestilliert. Man erhält 677 g rückführbares Biphenyl. Die Destillation wird dann fortgesetzt. Das erhaltene Destillat wiegt 425 g.

### B) Hydrierung

87 g Cyclohexylbiphenyl, 87 g Terphenyl und 3 g Raney-Nickel werden in einem Autoklav bei 160°C und einem konstanten Druck von 10 bar Wasserstoff hydriert. Nach einer Aufnahme von 70 bar Wasserstoff wird die Hydrierung beendet. Man saugt warm vom Katalysator ab und erhält 170 g der erfindungsgemäßen Zusammensetzung mit einer kinematischen Viskosität von 81 mm²/sec bei 20°C. Sie enthält:

| | |
|---|---|
| 2-Cyclohexylbiphenyl: | 13,1 % |
| 3-Cyclohexylbiphenyl: | 6,2 % |
| 4-Cyclohexylbiphenyl: | 19,1 % |
| Gemisch aus Dicyclohexylbenzolen, Phenylbicyclohexylen, Tercyclohexylen und höheren polycyclischen Kohlenwasserstoffen: | 61,6 % |

### Beispiel 2

### A) Herstellung von Cyclohexylbiphenylen

In einen Dreihalskolben mit Wasserabscheider werden 653 g Biphenyl und 12,5 g Tonsil KSFO vorgelegt. Bei 160°C läßt man innerhalb von 5 Stunden 214 g Cyclohexanol, welches zur Erstarrungspunkterniedrigung 1 % Wasser enthält, eintropfen. Das entstandene Reaktionswasser (ca. 39 ml) geht mit etwas zwischenzeitlich gebildetem Cyclohexen azeotrop über und wird ausgekreist. Man läßt 5 Stunden bei 180°C nachrühren. Das entstandene Rohprodukt (einschließlich Bleicherde) wiegt 841 g.

Diesem Rohprodukt setzt man bei 140°C 410 g rohes, schwefelhaltiges Terphenyl zu und rührt bei dieser Temperatur 1/2 Stunde. Das anschließende Absaugen erfolgt bei 130 bis 140°C.

In einer Destillation über eine 20 cm Kolonne wird überschüssiges Biphenyl abdestilliert., Man erhält bis zu einem Übergang von Kp._{20Torr} /165°C 385 g rückführbares Biphenyl. Der Destillationsrückstand wiegt 819 g und wird der Hydrierung zugeführt.

### B) Hydrierung

174 g des Gemisches aus Ansatz A und 8 g feuchtes Raney-Nickel werden in einen 0,7 l Autoklaven gegeben. Aus einem 0,7 l Vorratsgefäß mit 100 bar Wasserstoff wird über ein Reduzierventil der Hydrierdruck konstant bei 10 bar gehalten. Die Temperatur beträgt 180°C. Die Hydrierung wird beendet, wenn aus dem Vorratsgefäß 75 bar Wasserstoff entnommen sind. Dafür ist eine Zeit von 150 Minuten erforderlich. Nach dem Absaugen des Nickels erfolgt die Enddestillation des Filtrats über eine Destillationsbrücke:

| | |
|---|---|
| Kp._{0,5 Torr} 115 - ca. 220°C: | 164 g |
| Rückstand: | 9,5 g |

Das erhaltene Destillat ist dünnflüssig, klar und toluollöslich; es besitzt eine kinematische Viskosität von 89 mm²/sec bei 20°C. Es enthält:

| | |
|---|---|
| 2-Cyclohexylbiphenyl: | 16,9 % |
| 3-Cyclohexylbiphenyl: | 5,6 % |
| 4-Cyclohexylbiphenyl: | 14,5 % |
| Gemisch aus Dicyclohexylbenzolen, Phenylbicyclohexylen,Tercyclohexylen und höheren, polycyclischen Kohlenwasserstoffen: | 63,0 % |

## Patentansprüche

1. Mischungen aus
A. 4 bis 30 Gew.-% 2-Cyclohexylbiphenyl,
B. 1 bis 15 Gew.-% 3-Cyclohexylbiphenyl,
C. 4 bis 30 Gew.-% 4-Cyclohexylbiphenyl und
D. 45 bis 65 Gew.-% Dicyclohexylbenzolen und/oder Phenylbicyclohexylen und/oder Tercyclohexylen und gegebenenfalls höheren polycyclischen Kohlenwasserstoffen,
wobei sich die Prozentangaben jeweils auf die Summe der Komponenten A bis D beziehen.

2. Mischungen nach Anspruch 1 aus
10 bis 25 Gew.-% A,
3 bis 9 Gew.-% B,
11 bis 20 Gew.-% C und
45 bis 65 Gew.-% D.

3. Verfahren zur Herstellung von Mischungen nach Ansprüchen 1 und 2 durch partielle Hydrierung eines Gemisches aus Terphenylen und Cyclohexylbiphenylen, dadurch gekennzeichnet, daß die für die Hydrierung eingesetzte Mischung oder ihre Komponenten mit Bleicherde behandelt worden ist/sind.

4. Verfahren zur Herstellung von Mischungen nach Ansprüchen 1 und 2 durch partielle Hydrierung eines Gemisches aus Terphenylen und Cyclohexylbiphenylen in Gegenwart eines Hydrierkatalysators, dadurch gekennzeichnet, daß man die Hydrierung bei Temperaturen von 120 bis 190°C und Drucken von 2 bis 25 bar durchführt.

5. Verfahren nach Anspruch 4, wonach man die Hydrierung bei Temperaturen von 160 bis 180°C und bei Drucken von 8 bis 15 bar durchführt.

6. Verfahren zur Herstellung von Mischungen nach Ansprüchen 1 und 2, wonach man Biphenyl und Cylohexanol im Molverhältnis (1 bis 5):1 in Gegenwart von 0,3 bis 5 Gew.-%, bezogen auf eingesetztes Biphenyl, Bleicherde auf eine Temperatur von 140 bis 220°C erhitzt, wobei das freiwerdende Wasser zusammen mit einem Teil des entstandenen Cyclohexen azeotrop entfernt und das Cyclohexen rückgeführt wird, dem entstandenen Reaktionsgemisch das 0,5 bis 1,2-fache der im Reaktionsgemisch befindlichen Cyclohexylbiphenyle an Terphenyl und so viel Biphenyl zusetzt, daß der Anteil Biphenyl im Rohgemisch (nach der Terphenylzugabe) mindestens 30 Gew.-% beträgt, und die resultierende Mischung bei einer Temperatur von 130 bis 160°C 0,5 bis 4 Stunden rührt, die Bleicherde und Biphenyl abtrennt, den Rückstand in Gegenwart eines Hydrierkatalysators bei einer Temperatur von 120 bis 190°C und einem Druck von 2 bis 25 bar hydriert, bis der Anteil D der anspruchsgemäßen Mischungen im Bereich von 45 bis 65 Gew.-%, bezogen auf die Komponenten A bis D, liegt, den Hydrierkatalysator abtrennt und das verbleibende Reaktionsgemisch destilliert.

7. Verfahren nach Anspruch 6, wonach man 0,8 bis 2 Gew.-% Bleicherde, bezogen auf eingesetztes Biphenyl, verwendet.

8. Verwendung der Mischungen nach Ansprüchen 1 und 2 als Wärmeträger.

## Claims

1. Mixtures of
A. 4 to 30 % by weight of 2-cyclohexylbiphenyl,
B. 1 to 15 % by weight of 3-cyclohexylbiphenyl,
C. 4 to 30 % by weight of 4-cyclohexylbiphenyl and
D. 45 to 65 % by weight of dicyclohexylbenzenes and/or phenylbicyclohexyls and/or tercyclohexyls and optionally higher polycyclic hydrocarbons,
the percentage data in each case relating to the sum of the components A to D.

2. Mixtures according to Claim 1, of
10 to 25 % by weight of A,
3 to 9 % by weight of B,
11 to 20 % by weight of C and
45 to 65 % by weight of D.

3. Process for the preparation of mixtures according to Claims 1 and 2, by partial hydrogenation of a mixture of terphenyls and cyclohexylbiphenyls, characterised in that the mixture used for the hydrogenation, or its components, is/are treated with bleaching earth.

4. Process for the preparation of mixtures according to Claims 1 and 2 by partial hydrogenation of a mixture of terphenyls and cyclohexylbiphenyls in the presence of a hydrogenation catalyst, characterised in that the hydrogenation is carried out at temperatures of 120 to 190°C, and under pressures of 2 to 25 bar.

5. Process according to Claim 4, according to which the hydrogenation is carried out at temperatures of 160 to 180°C and under pressures of 8 to 15 bar.

6. Process for the preparation of mixtures according to Claims 1 and 2, according to which process biphenyl and cyclohexanol are heated in a molar ratio of (1 to 5):1 in the presence of 0.3 to 5 % by weight, based on biphenyl employed, of bleaching earth to a temperature of 140 to 220°C, in which process the water liberated is removed as an azeotrope together with a portion of the cyclohexene formed and the cyclohexene is recycled, terphenyl, in 0.5 to 1.2 times the amount of cyclohexylbiphenyls present in the reaction mixture, and biphenyl, in an amount such that the proportion of biphenyl in the crude mixture (after the addition of terphenyl) is at least 30 % by weight, are added to the resulting reaction mixture and the resulting mixture is stirred at a temperature of 130 to 160°C for 0.5 to 4 hours, the bleaching earth and biphenyl are separated off, the residue is hydrogenated in the presence of a hydrogenation catalyst at a temperature of 120 to 190°C and under a pressure of 2 to 25 bar until the proportion D in the mixtures according to the claim is in the range of 45 to 65 % by weight, based on the components A to D, the hydrogenation catalyst is separated off and the residual reaction mixture is distilled.

7. Process according to Claim 6, according to which 0.8 to 2 % by weight of bleaching earth, based on biphenyl employed, are used.

8. Use of the mixtures according to Claims 1 and 2 as heat transfer media.

## Revendications

1. Mélanges constitués
A. de 4 à 30 % en poids de 2-cyclohexylbiphényle,
B. de 1 à 15 % en poids de 3-cyclohexylbiphényle,
C. de 4 à 30 % en poids de 4-cyclohexylbiphényle,
D. de 45 à 65 % en poids de dicyclohexylbenzènes et/ou de phénylbicyclohexyles et/ou de tercyclohexyles et éventuellement d'hydrocarbures polycycliques supérieurs,
les données en pour-cent se rapportant à chaque fois à la somme des constituants A à D.

2. Mélanges selon la revendication 1, constitués
de 10 à 25 % en poids de A,
de 3 à 9 % en poids de B,
de 11 à 20 % en poids de C et
de 45 à 65% en poids de D.

3. Procédé pour la préparation de mélanges selon les revendications 1 et 2 par hydrogénation partielle d'un mélange constitué de terphényles et de cyclohexylbiphényles, caractérisé en ce que le mélange ou ses constituants utilisé(s) pour l'hydrogénation a été/ont été traités avec de l'argile à smectite.

4. Procédé pour la préparation de mélanges selon les revendications 1 et 2 par hydrogénation partielle d'un mélange constitué de terphényles et de cyclohexylbiphényles en présence d'un catalyseur d'hydrogénation, caractérisé en ce qu'on réalise l'hydrogénation à des températures de 120 à 190°C et à des pression de 2 à 25 bars.

5. Procédé selon la revendication 4, d'après lequel on réalise l'hydrogénation à des températures de 160 à 180°C et à des pressions de 8 à 15 bars.

6. Procédé pour la préparation de mélanges selon les revendications 1 et 2, d'après lequel on chauffe du biphényle et du cyclohexanol dans un rapport molaire (1 à 5):1 en présence de 0,3 à 5 % en poids, rapportés au biphényle utilisé, d'argile à smectite, à une température de 140 à 220°C, l'eau libérée étant éliminée de manière azéotrope avec une partie du cyclohexène produit et le cyclohexène étant recyclé, on ajoute au mélange réactionnel produit 0,5 à 1,2 fois la quantité des cyclohexylbiphényles se trouvant dans le mélange réactionnel en terphényle et autant en biphényle, de sorte que la part en biphényle dans le mélange brut (après l'addition de terphényle) est au moins égale à 30 % en poids, et on agite le mélange résultant à une température de 130 à 160°C pendant 0,5 à 4 h, on sépare l'argile à smectite et le biphényle, on hydrogène le résidu en présence d'un catalyseur d'hydrogénation à une température de 120 à 190°C et à une pression de 2 à 25 bars jusqu'à ce que la part D des mélanges revendiqués se trouve dans le domaine de 45 à 65 % en poids, rapportés à la somme des constituants A à D, on sépare le catalyseur d'hydrogénation et on distille le mélange réactionnel restant.

7. Procédé selon la revendication 6, d'après lequel on utilise de 0,8 à 2 % en poids d'argile à smectite, rapportés au biphényle utilisé.

8. Utilisation des mélanges selon les revendications 1 et 2 en tant qu'agents caloporteurs.
